# EUROPEAN PATENT SPECIFICATION

(11) **EP 0 484 240 B1**
(45) Date of publication and mention of the grant of the patent: **02.01.1997**
(21) Application number: 91402911.1
(22) Date of filing: 30.10.1991
(51) Int. Cl.: C12P 3/00

(54) **Process for preparing hydrogen gas using microorganism**
Verfahren zur Herstellung von Hydrogengas unter Verwendung von Mikroorganismen
Procédé pour la préparation de gaz hydrogène en utilisant des micro-organismes

(30) Priority: 02.11.1990 JP 295384/90
(43) Date of publication of application: 06.05.1992
(73) Proprietor: Kajima Corporation, Tokyo 107 (JP)
(72) Inventor: Taguchi, Fumiaki, Sagamihara-shi, Kanagawa-ken (JP); Moritomo, Masayoshi, Tokyo (JP); Kyoya, Takeshi, Kamakura-shi, Kanagawa-ken (JP); Takano, Mikio, Tokyo (JP)
(74) Representative: Clisci, Serge

(56) References cited:
- APPLIED & ENVIRONMENTAL MICROBIOLOGY, vol. 35, no. 5, May 1978, Washington, DC (US); J.E. SCHULTZ et al., pp. 930-936/
- APPLIED & ENVIRONMENTAL MICROBIOLOGY, vol. 53, no. 4, April 1987, Washington, DC (US); S.F. HIU et al., pp. 697-703/
- CHEMICAL ABSTRACTS, vol. 103, no. 19, November 1985, Columbus, OH (US); AGENCY OF INDUSTRIAL SCIENCES & TECHNOLOGY, p. 587, no. 159129r/
- INTERNATL. JOURNAL OF HYDROGEN ENERGY, vol. 12, no. 9, 1987, Oxford (GB); S. TANISHO et al., pp. 623-627/

## Description

### BACKGROUND OF THE INVENTION

### FIELD OF THE INVENTION

The present invention relates to a process for preparing hydrogen gas and more particularly, a process for preparing hydrogen gas on an industrial scale, using a novel microorganism. The present invention further provides a process for preparing hydrogen gas using a microorganism which uses as a substrate polysaccharides containing a glucose unit as a constituent.

The microorganisms used in the present invention are excellent in productivity of hydrogen gas and thus useful for industrial production of hydrogen gas. Therefore, the present invention greatly contributes to the technical field of energy. In addition, the microorganisms used in the present invention also possess the ability of degrading various sugars strongly in a wide range of sugar kinds and are thus useful also for treatment of waste water discharged in food industry or paper making industry or waste water discharged in everyday life, especially waste water containing large amounts of sugars. Therefore, the present invention greatly contributes also to the technical field of waste water treatment or pollution prevention.

### DESCRIPTION OF THE PRIOR ART

In the current industrial society, fossil fuel such as petroleum, coal, natural gas, etc. has been consumed in huge amounts and the fossil fuel discharges large quantities of NOx, SOx and CO₂, etc. by combustion. As a result, various problems such as environmental pollution, acid rain, greenhouse effect of the earth, etc. are caused. Furthermore, it is said that fuel deposits are limited and are dried up in the near future, resulting in important social problems.

resources Under these situations, new clean energy resources free of environmental pollution as a substitute for fossil fuel have been desired worldwide. As energy resources in the next generation in place of petroleum, attention has been currently focused on alcohol and methane gas. However, alcohol and methane gas are still problematic in producing large amounts of CO₂ upon combustion.

Now attention has been brought to hydrogen gas. In hydrogen gas, exothermic energy per unit weight by combustion is 3 times that of petroleum and its by-product is H₂O alone. It is thus expected that hydrogen gas would be an ideal clean energy source in the next generation.

However, the industrial production of hydrogen gas is actually carried out by means of electrolysis of water, high pressure thermal decomposition of natural gas, etc. These methods require fossil fuels as their energy resources. Unless the problems of energy sources in these methods are solved, the various problems described above including environmental pollution are not principally solved.

Now attention was paid to microorganisms and severaL studies on production of hydrogen gas by microorganisms were made. Certainly if a method for producing-hydrogen gas by microorganism is established, the method would involve advantages that the system is easily constructed and energy consumption is extremely small, since the reaction is carried out at normal temperature under normal pressure. Furthermore, reproducible biomass is used as a raw material for producing hydrogen gas and this biomass is originally a conversion of solar energy. Therefore, the use of biomass is an effective utilization of natural energy. Furthermore, the production of hydrogen gas by microorganism has an advantage that environmental pollution is solved due to efficient treatment of waste water since it is generally possible to use as a raw material an organic substance present in wastes or waste water.

As described above, several studies were made on the production of hydrogen gas by microorganisms and as a result, some microorganisms capable of producing hydrogen gas were found. These known microorganisms capable of producing hydrogen gas are roughly classified into photosynthetic microorganisms and non-photosynthetic bacteria. The former includes Rhodobacter sphaeroides which is a photosynthetic bacterium and Oscillatoria sp. Miami BG7 which is a blue green algae. The latter includes Azotobacter chroococuum and Klebsiella pneumoniae which are nitrogen fixing bacteria, Escherichia coli and Enterobacter aerogenes which are facultative anaerobic bacteria, and Clostridium butyricum which is an anaerobic bacterium; and the like.

Indeed, the production of hydrogen gas by microorganism has such extremely excellent advantages as a method for preparing hydrogen gas; however, it is the actual situation that is too far from to establishing such a process on an industrial scale. In particular, any microorganisms having such a high productivity of hydrogen gas that make industrial production possible has not yet been found in the studies made so far.

As described above, the technique for producing hydrogen gas by microorganisms has not reached yet the industrial level.

Any of microorganisms known so far provides not only a poor productivity of hydrogen gas but also requires a fermenter having a wide surface area and large quantities of water for producing hydrogen gas with the photosynthetic microorganisms among these microorganisms, since solar energy is utilized.

On the other hand, it is possible to produce hydrogen gas by non-photosynthetic bacteria even in a fermenter of small scale. Such a fermenter may be installed under the ground and therefore, there is a wide selection for the place to install the fermenter. It is thus considered that the use of non-photosynthetic bacteria is more advantageous than that of photosynthetic microorganisms for producing hydrogen gas.

Among the microorganisms isolated so far, the microorganism capable of producing hydrogen gas most efficiently is considered to be the Enterobacter aerogenes E82005 strain (Tanisho S., et al., Int. J. Hydrogen Energy, 12, 623, 1987; Biochim. Biophys. Acta, 973, 1, 1989). However, this strain is a facultative anaerobic bacterium. The strain grows also under anaerobic conditions but grows more actively under aerobic conditions. Therefore, when large quantities of hydrogen are produced in a fermenter, it is difficult to maintain aerobic conditions in the fermenter ; consequently the use of this strain is not suitable for an industrial production of hydrogen gas.

The article by SCHULTZ et al., Applied and Environmental Microbiology, 35 (No. 5), 930-936. (1978), discloses several microorganisms isolated from hindguts of termites, but does not mention the two strains according to this invention, namely the Clostridium beijerinckii FERM BP-3592 strain and the anaerobic asporogenic bacterium FERM BP-3593 strain.

The article by HIU S.F. et al., Applied and Environmental Microbiology, 53 (No. 4), 697-703, (1987), is concerned with Clostridium butylicum strains (also called or similar to Clostridium beijerinckii strains) which produce alcohol. To be precise. it discloses two reference strains : Clostridium butylicum NRRL B592 and Clostridium butylicum NRRL B593. which are different from the two strains according to this invention.

Chemical Abstracts 103 : 159129r (1985), which sums up the Japanese publication JP-A-60 75 288, relates to the production of hydrogen by Clostridium, in particular Clostridium butylicum IFO 13949, a strain which is different from the FERM BP-3592 strain of this invention.

### SUMMARY OF THE INVENTION

An object of the present invention is to newly isolate hydrogen gas-producing bacteria which can utilize not only glucose but also polysaccharide as substrate and are suitable for industrial production. Another object of the present invention is to industrially establish an excellent system for producing hydrogen gas, by newly isolated microorganisms capable of efficiently treating waste water, especially waste water abundant in sugar-containing plants.

The present invention has been made to achieve the objects described above. In spite of extensive investigations from various aspects, no successful results were obtained according to the prior art teaching. Therefore, the conventional concept should have been greatly switched for screening a microorganism which can achieve the objects.

Thus, the present inventors have made extensive investigations on the source of microorganisms and as a result, have sought the source of hydrogen gas-producing bacteria for insects and finally succeeded in isolating novel bacteria having an extremely fast reaction velocity and capable of producing large quantities of hydrogen gas, from Termites formosans. Furthermore, it has also been surprisingly confirmed that these microorganisms can not only assimilate glucose but also have an extremely high ability of degrading polysaccharide containing glucose as a constituent, namely, can directly produce hydrogen gas using as substrate not only monosaccharide such as glucose, etc. but also polysaccharide containing the same as a constituent; and that such polysaccharide-containing matters, for example, organic waste water can be effectively degraded and decomposed. Based on these effective new findings, the present invention has been accomplished.

That is, as a result of extensive investigations, the present inventors have succeeded in isolating new hydrogen gas-producing bacteria which can directly produce hydrogen gas using as raw materials not only sugars alone but also starch. Therefore, the present invention has finally been accomplished.

### SUBJECT OF THE INVENTION

According to a first aspect of this invention, is provided a process for preparing hydrogen gas comprising culturing Clostridium beijerinckii FERM BP-3592 in a medium containing glucose and/or polysaccharide containing a glucose unit for a time sufficient to produce hydrogen gas and recovering the hydrogen gas.

According to a second aspect of this invention. is provided a process for preparing hydrogen gas comprising culturing anaerobic asporogenic bacterium strain, FERM BP-3593, in a medium containing glucose and/or polysaccharide containing a glucose unit for a time sufficient to produce hydrogen gas and recovering the hydrogen gas.

According to a further aspect of this invention, are provided a biologically pure culture of Clostridium beijerinckii FERM BP-3592, and a biologically pure culture of bacterium strain FERM BP-3593.

### DETAILED DESCRIPTION OF THE PREFERRED EMBODIMENTS

For practising the present invention, it is required to isolate anaerobic bacteria from Termites formosans. For isolation, for example, Termites formosans is suffocated in the living state and the desired microorganism is isolated by using a medium for isolation containing acetic acid salt and formic acid salt [preferred salts are alkali (or alkaline earth) metal salts].

For example, the present inventors devised a medium (hereafter abbreviated as 1/50 N⁺) obtained by adding 2.5 g/l each of sodium acetate and sodium formate which are substrate of methane gas and substrate of hydrogen gas, respectively, to a medium which is a dilution of nutrient broth (manufactured by Nissui Pharmaceutical Co., Ltd.) to 1/50 (hereafter abbreviated as 1/50 N), and used the thus obtained medium as the medium for culturing and isolating the bacteria. The inventors also devised oligotrophic 1/50 N⁺ agar medium obtained by supplementing agar to this 1/50 N⁺ in a rate of 1.5%. Then taking into account that the present invention will be applied to waste water treatment of energy recovery type in the future, anaerobic incubation is used at 35°C.

In order to efficiently isolate anaerobic bacteria from white ants or Termites formosans and incubate, it is attempted to avoid contact with oxygen as possible. For this reason, white ants which are taken out of the nest and are alive are put in a Petri dish, are encased in an anaerobic incubator (Glove Box, manufactured by Forma Co., U.S.A.) and suffocated under atmosphere in an anaerobic gaseous mixture (10% of H₂, 10% of CO₂ and 80% of N₂). Without mashing white ants, ten ants are added to 20 ml of 1/50 N⁺ agar medium. After thoroughly mixing, the mixture is solidified. By this procedure, white ants are in such a state that the ants are embedded in agar. Thus, the condition that the ants hardly contact even with the gaseous mixture is provided.

Ten sheets of the agar plate are incubated at 35°C for 3 weeks in the anaerobic gaseous mixture.

Then, by the procedures for bacteriological isolation, 153 strains are isolated and the hydrogen gas productivity of the 153 strains is presumed by screening. As the result, it has been found that 141 strains corresponding to 93% can produce hydrogen gas. Then, oxygen auxotrophy test is carried out. As the result, 8 strains are facultative anaerobic bacteria. Finally, the inventors have succeeded in isolating 2 candidates, namely AM21B and AM37F, suited for the purpose.

Among the microorganisms isolated to date, Enterobacter aerogenes E82005 strain is believed to produce hydrogen gas most efficiently, as has been described above. This strain produces 11 mmol H₂/ℓ-medium.hr or 246 ml H₂/ℓ-medium.hr, whereas the two strains out of the strains isolated by the inventors have a hydrogen gas productivity which is much higher than the known strain, as will be later described.

Based on the properties of these strains (according to API 20A, Rap ANA II system), AM21B is identified to be Clostridium beijerinckii and AM37F is quite a new hydrogen-producing bacterium ; it is impossible to identify. In more detail, any bacterium that is gram positive, rod, asporogenic anaerobic and capable of producing hydrogen gas has not been reported at all and is quite novel.

starch

The present inventors have made investigations on starch degradation ability and gas productivity of the novel bacteria described above, and have succeeded in providing the process of the present invention for preparing hydrogen gas using this novel bacteria.

The present invention provides the process for preparing hydrogen gas which comprises culturing the hydrogen gas-producing bacteria, using monosaccharide substrate and/or using as substrate polysaccharides such as disaccharide or higher sugar containing a glucose unit as a substrate. As the hydrogen gas-producing bacteria, AM21B or AM37F described above are used.

AM21B and AM37F strains have been deposited in Fermentation Research Institute of the Agency of Industrial Science and Technology in Japan under FERM BP-3592 and FERM BP- 3593, respectively.

General properties, biochemical properties, enzyme activity and hydrogen gas productivity of AM21B and AM37F are shown in Tables 1, 2, 3 and 4.

**Table 1**

| General Property | | | | | | | |
|---|---|---|---|---|---|---|---|
| | | | | | | Growth Ability | |
| Strain | Gram Staining | Shape | Motility | Spore | Oxygen Auxotrophy | Peptone Water | Glucose-Added Peptone Water |
| AM21B | positive | rod | motile | yes | no* | - | + |
| AM37F | positive | rod | motile | no | no* | - | + |

| | | | | | | | |
|---|---|---|---|---|---|---|---|
| * anaerobic | | | | | | | |

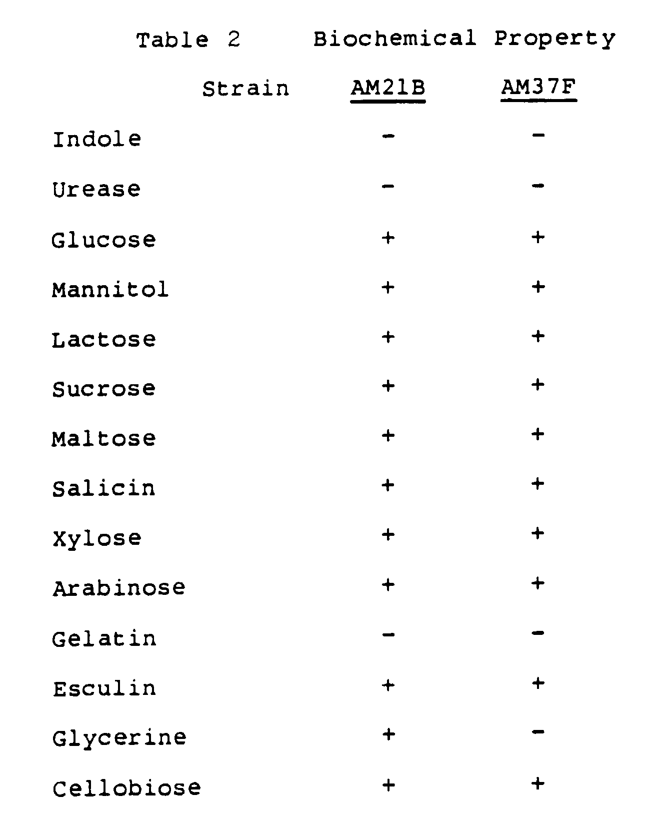

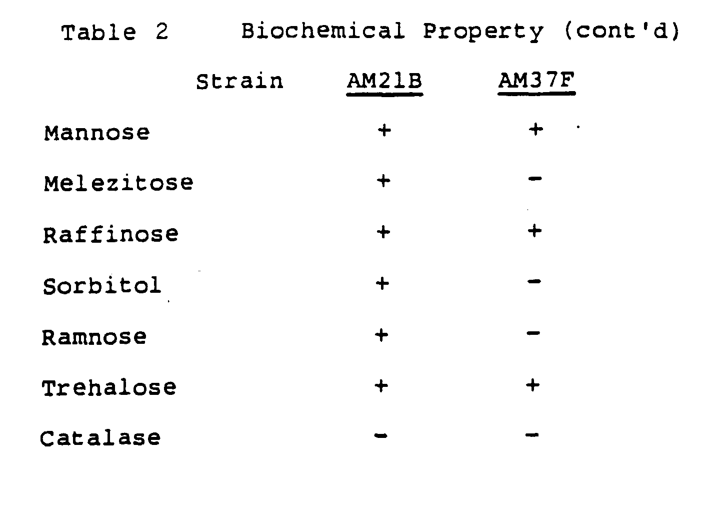

**Table 3**

| Enzyme Activity | | |
|---|---|---|
| Strain | AM21B | AM37F |
| α-arabinosidase | - | - |
| β-galactosidase | - | - |
| α-glucosidase | + | ++++ |
| β-glucosidase | + | - |
| α-galactosidase | - | ++++ |
| α-fucosidase | - | - |
| N-acetylglucosaminidase | - | - |
| alkaline phosphatase | - | - |
| leucylglycine aminopeptidase | - | - |
| glycine aminopeptidase | - | - |
| proline aminopeptidase | - | - |
| phenylalanine aminopeptidase | - | - |
| arginine aminopeptidase | - | - |
| serine aminopeptidase | - | - |
| pyrrolidone aminopeptidase | - | - |
| tryptophanase | - | - |

**Table 4**

| Strain | Hydrogen Gas Productivity (ml H₂/ℓ.hr) | |
|---|---|---|
| E. aerogenes E82005 * | 246 ml | 1.0 |
| | | (index for producing hydrogen gas) |
| AM37F | 2,790 | 11.3 |
| AM21B | 2,515 | 10.2 |

| | | |
|---|---|---|
| * Tanisho S., Int. J. Hydrogen Energy 12: 623 1987 " Biochim. Biophys. Acta, 973, 1, 1989 | | |

As stated above, Enterobacter aerogenes E82005 strain showed hydrogen gas productivity of 246 ml/ℓ.hr, whereas the hydrogen gas productivity of the two strains which were successfully isolated from white ants by the present inventors reached 10 to 11 times that of the E82005 strain, indicating that the strains are usable sufficiently for the purpose of industrial use.

Hereafter the present invention is described by referring to the examples.

### Example 1

Carbon source-free PY liquid medium [1% peptone, manufactured by Eiken Chemical Co., 0.5% yeast extract, manufactured by Nissui Pharmaceutical Co.] and PYG or PYS liquid medium obtained by supplementing glucose or starch (manufactured by Difco) as a carbon source to PY liquid medium in a concentration of 0.5% were prepared. Each medium was separately charged by 3 ml each in a small test tube with a Dahram tube and sterilized in an autoclave. After it was confirmed that no bubble remained in the Dahram tube, AM21B or AM37F was inoculated on each medium and cultured in an anaerobic incubator (in an anaerobic gaseous mixture composed of 10% CO₂, 10% H₂ and 80% N₂) at 36°C overnight, whereby growth of the bacteria and the presence or absence of bubble in the Dahram tube were observed. Where gas remained in the Dahram tube, it was assumed that hydrogen gas was present by an explosion in combustion test.

Assimilability of starch as the carbon source was tested. The results shown in Table 5 were obtained. The novel strains obtained by the present inventors did not grow in peptone alone but grew well when glucose or yeast extract was supplemented to peptone. When glucose or starch was added to PY medium, it was noted that all bacteria tested grew well and at the same time, produced gas (hydrogen gas) which burned with an explosion. With respect to bacteria which hydrolyze starch, many reports have been made so far but any bacteria capable of producing hydrogen gas directly from starch has not been heretofore reported and this is the first report.

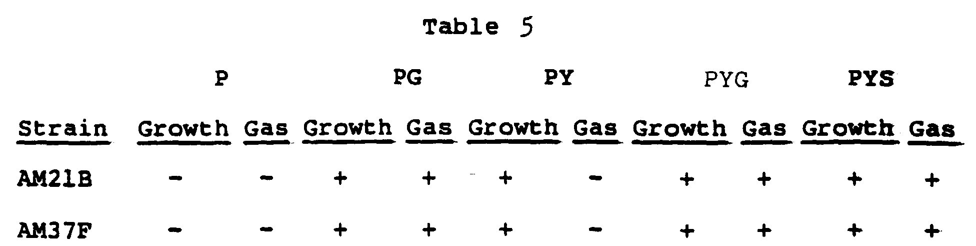

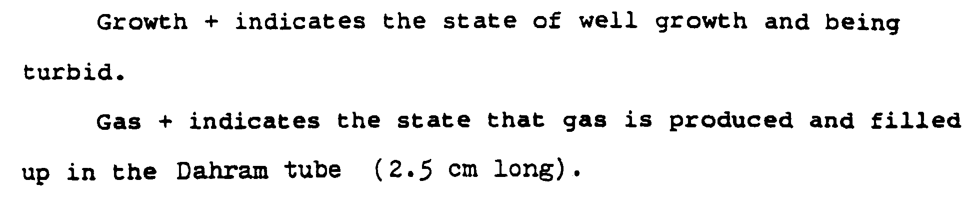

### Example 2

GAM bouillon medium containing 0.3% glucose and 0.5% starch, PYG medium containing 1.0% glucose and PYS medium containing 1.0% starch were prepared by 100 ml each. AM21B or AM37F was inoculated on each medium, respectively, whereby the gas produced was measured. The composition of gas was analyzed by gas chromatography.

The results are shown in Table 6. It was confirmed that both AM21B and AM37F strains produced hydrogen gas from starch, almost on the same level as from glucose.

**Table 6**

| | Additive (%) | | | | | |
|---|---|---|---|---|---|---|
| Strain | Glucose | Starch | Total Gas Produced (ml) | Rate of Gas Produced (ml/hr) | Hydrogen Gas (ml) | Rate of Hydrogen Gas Produced (ml/hr) |
| AM21B GAM | 0.3 | 0.5 | 2900 | 850 | 1200 | 340 |
| PYG | 1.0 | 0 | 3000 | 650 | 1000 | 210 |
| PYS | 0 | 1.0 | 3000 | 585 | 900 | 180 |
| AM37F GAM | 0.3 | 0.5 | 2800 | 820 | 1150 | 336 |
| PYG | 1.0 | 0 | 2600 | 610 | 860 | 200 |
| PYS | 0 | 1.0 | 2400 | 480 | 720 | 150 |

### Example 3

GAM bouillon ≠1 (containing 0.3% glucose and 0.5% starch), GAM bouillon #2 containing 1% glucose prepared by supplementing 0.7% glucose to GAM bouillon ≠1 and medium PY #3 to PY #7 by supplementing glucose or starch to PY medium in an appropriate proportion were prepared by 100 ml each. AM21B strain was inoculated on each medium followed by spinner culture in a thermostat at 36°C. The amount of gas evolved was measured. Based on the concentration of hydrogen gas obtained by the compositional analysis of the gas, the amount of hydrogen gas produced was calculated. The results are shown in Table 7.

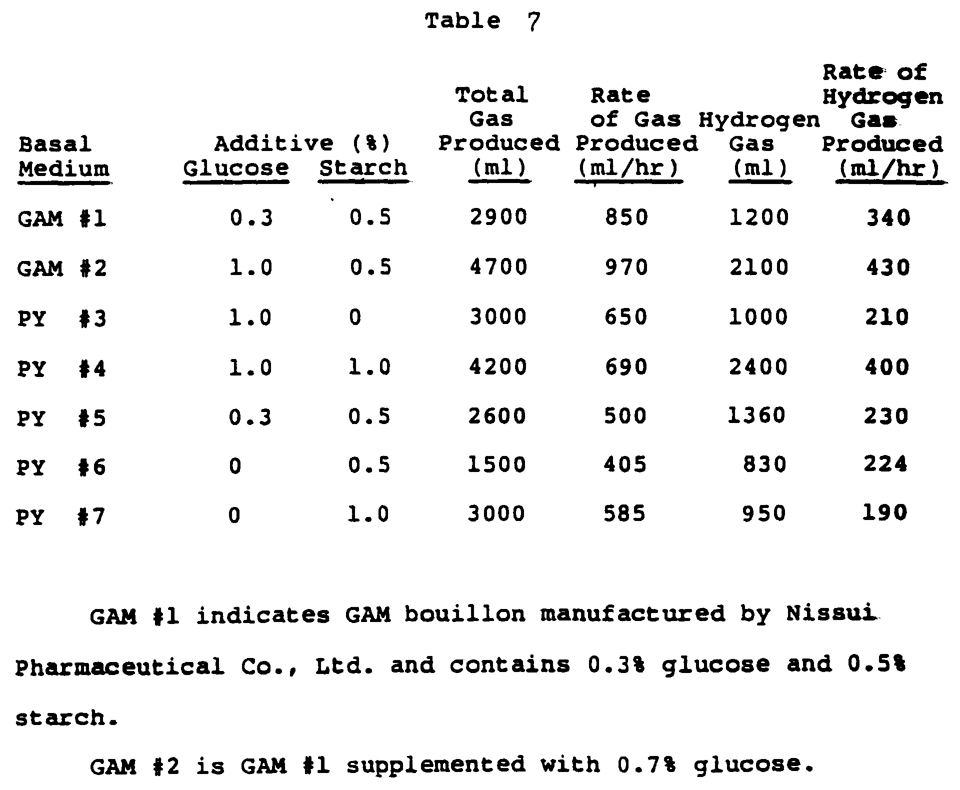

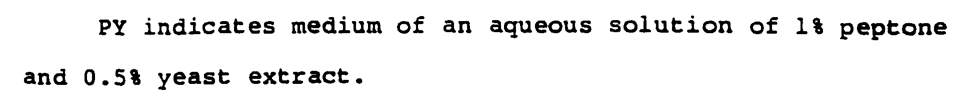

As is clear from Table 7 described above, AM21B strain produced 1500 ml and 3000 ml of gas, respectively, in PY #6 and PY #7 containing starch alone. It was confirmed that when the concentration became double (from 0.5% to 1%), the amount of gas produced was doubled. From GAM #1 and PY #5, the amounts of gas produced, 2900 ml and 2600 ml close to each other, were noted, respectively. From PY #3 and PY #7, 3000 ml of the gas was produced, respectively. It was thus confirmed that the new strains described above had reactivity with extremely excellent efficiency in producing hydrogen gas from starch.

As is evident from these results, hydrogen gas can be produced in large quantities not only from monosaccharide as substrate but also from polysaccharide, directly in large quantities. It is not too much to say that the method for industrial production of hydrogen gas using microorganism has been established by the present invention for the first time.

These results also reveal that in treating natural matters containing sugar or composed of sugar, these strains are very useful. The strains may be utilized also for clarification of juice or various agricultural products, reduction in viscosity and increasing fluidity. These strains are especially useful for treating organic waste waters discharged from beet factories, or orange processing factories and other waste waters containing organic substances such as fibers, starch, and polysaccharides. It is also possible to apply the present invention to environmental purification by treating waste waters and wastes.

## Claims

1. A process for preparing hydrogen gas comprising culturing Clostridium beijerinckii FERM BP-3592 in a medium containing glucose and/or polysaccharide containing a glucose unit for a time sufficient to produce hydrogen gas and recovering the hydrogen gas.

2. A process for preparing hydrogen gas comprising culturing anaerobic asporogenic bacterium strain FERM BP-3593 in a medium containing glucose and/or polysaccharide containing a glucose unit for a time sufficient to produce hydrogen gas and recovering the hydrogen gas.

3. The process according to claim 1 wherein the polysaccharide containing a glucose unit is starch.

4. The process according to claim 2 wherein the polysaccharide containing a glucose unit is starch.

5. The process according to claim 1 wherein said culturing is conducted at a temperature of 36°C.

6. The process according to claim 2 wherein said culturing is conducted at a temperature of 36°C.

7. A biologically pure culture of Clostridium beijerinckii FERM BP-3592.

8. A biologically pure culture of bacterium strain FERM BP-3593.

## Patentansprüche

1. Verfahren zum Herstellen von Wasserstoffgas, das den Schritt des Kultivierens von Clostridium beijerinckii FERM BP-3592 in einem Glucose und/oder Polysaccharid mit einer Glucoseeinheit enthaltendem Medium für einen Zeitraum ausreichend zum Erzeugen von Wasserstoffgas und den weiteren Schritt des Gewinnens des Wasserstoffgases umfaßt.

2. Verfahren zum Herstellen von Wasserstoffgas, das den Schritt des Kultivierens des aneroben asporogenen Bakterienstamms FERM BP-3593 in einem Glucose und/oder Polysaccharid mit einer Glucoseeinheit enthaltendem Medium für einen Zeitraum ausreichend zum Erzeugen von Wasserstoffgas und den weiteren Schritt des Gewinnens des Wasserstoffgases umfaßt.

3. Verfahren nach Anspruch 1, bei dem das eine Glucoseeinheit enthaltende Polysaccharid Stärke ist.

4. Verfahren nach Anspruch 2, bei dem das eine Glucoseeinheit enthaltende Polysaccharid Stärke ist.

5. Verfahren nach Anspruch 1, bei dem das Kultivieren bei einer Temperatur von 36°C durchgeführt wird.

6. Verfahren nach Anspruch 2, bei dem das Kultivieren bei einer Temperatur von 36°C durchgeführt wird.

7. Eine biologisch reine Kultur von Clostridium beiierinckii FERM BP-3592.

8. Eine biologisch reine Kultur des Baktierenstamms FERM BP-3593.

## Revendications

1. Procédé de préparation d'hydrogène gazeux, qui consiste à cultiver Clostridium beijerinckii FERM BP-3592 dans un milieu contenant du glucose et/ou un polysaccharide contenant une unité de glucose, pendant un laps de temps suffisant pour produire de l'hydrogène gazeux, et à récupérer l'hydrogène gazeux.

2. Procédé de préparation d'hydrogène gazeux, qui consiste à cultiver la souche bactérienne asporogène anaérobie FERM BP-3593 dans un milieu contenant du glucose et/ou un polysaccharide contenant une unité de glucose, pendant un laps de temps suffisant pour produire de l'hydrogène gazeux, et à récupérer l'hydrogène gazeux.

3. Procédé selon la revendication 1, dans lequel le polysaccharide contenant une unité de glucose est l'amidon.

4. Procédé selon la revendication 2, dans lequel le polysaccharide contenant une unité de glucose est l'amidon.

5. Procédé selon la revendication 1, dans lequel ladite culture est mise en oeuvre à une température de 36°C.

6. Procédé selon la revendication 2, dans lequel ladite culture est mise en oeuvre à une température de 36°C.

7. Culture biologiquement pure de Clostridium beijerinckii FERM BP-3592.

8. Culture biologiquement pure de la souche bactérienne FERM BP-3593.
